(19) Europäisches Patentamt
European Patent Office
Office européen des brevets

(11) Veröffentlichungsnummer: **0 414 062 A2**

(12) **EUROPÄISCHE PATENTANMELDUNG**

(21) Anmeldenummer: **90115355.1**

(22) Anmeldetag: **10.08.90**

(51) Int. Cl.$^5$: **C07C 63/331**, C07C 63/49, C07C 65/24, C07C 51/255

(30) Priorität: **23.08.89 DE 3927772**

(43) Veröffentlichungstag der Anmeldung: **27.02.91 Patentblatt 91/09**

(84) Benannte Vertragsstaaten: **DE FR GB IT**

(71) Anmelder: **BAYER AG**

**D-5090 Leverkusen 1 Bayerwerk(DE)**

(72) Erfinder: **Weider, Richard, Dr.**
**Quettingerstrasse 78**
**D-5090 Leverkusen 3(DE)**
Erfinder: **Scholl, Thomas, Dr.**
**Elbingerstrasse 33**
**D-4005 Meerbusch 2(DE)**

(54) **Verfahren zur Herstellung von aromatischen Dicarbonsäuren und neue aromatische Dicarbonsäuren.**

(57) Aromatische Dicarbonsäuren der Formel

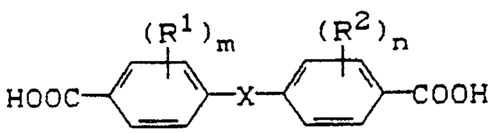

(I),

in der
$R^1$, $R^2$, m, n und X die in der Beschreibung genannte Bedeutung haben,
können aus den zugrunde liegenden Bisphenolen der Formel

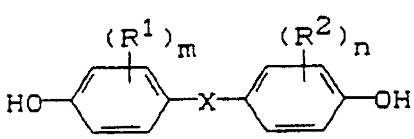

(II)

hergestellt werden, wenn man die Bisphenole zunächst zu den Bissulfonaten umsetzt, die Sulfonatgruppen katalytisch mit $H_2$ entfernt und die dabei erhaltenen Kohlenwasserstoffe in bekannter Weise zweifach acyliert und die Acylgruppen zu den Carboxylgruppen oxidiert.

Viele der so herstellbaren aromatischen Dicarbonsäuren sind neu.

EP 0 414 062 A2

# VERFAHREN ZUR HERSTELLUNG VON AROMATISCHEN DICARBONSÄUREN UND NEUE AROMATISCHE DICARBONSÄUREN

Aromatische Carbonsäuren vom Typ der Formel

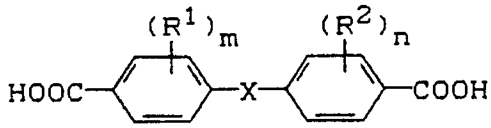

(I)

bzw. ihre Säurechloride und Ester sind vor allem wertvolle Zwischenprodukte auf dem Polymergebiet und eignen sich beispielsweise als Monomere zur Herstellung von Polyestern, Polyestercarbonaten oder Polyamiden mit hoher Wärmeformbeständigkeit.

Wegen der schlechten Zugänglichkeit sind jedoch nur wenige Vertreter dieses Typs bekannt. So wird in US 2.794.822 die Herstellung des 2,2-Bis-(p-carboxyphenyl)-propans nach folgenden Verfahren beschrieben: Zum einen wird Aceton mit Phosphorpentachlorid zum 2,2-Dichlorpropan (27 % Ausbeute) umgesetzt, welches mit Benzol in Ge genwart von Aluminiumchlorid weiter zum 2,2-Diphenylpropan reagiert (46 %). Dieser Kohlenwasserstoff wurde dann in para,para-Stellung mit Acetylchlorid und Aluminiumchlorid zweifach acetyliert (56 %) und das Diacetylprodukt mit Natriumhypochlorit zum 2,2-Bis-(p-carboxyphenyl)-propan als der aromatischen Dicarbonsäure oxidiert (89 %). Der andere dort beschriebene Weg führt, ebenfalls ausgehend vom 2,2-Dichlor-propan, mit Toluol in Gegenwart von Aluminiumchlorid zum 2,2-Bis-(p-tolyl)-propan und dessen Oxidation mit Salpetersäure in Gegenwart von Ammoniumvanadat als Katalysator zur genannten aromatischen Dicarbonsäure. Ein noch weiterer in US 2.794.822 beschriebener Weg führt über die Kondensation von Aceton mit Anilin zum 2,2-Bis-(p-aminophenyl)-propan, welches mit Hilfe von Natriumnitrit und Cyanidionen zum entsprechenden Dinitril umgesetzt wird, welches sodann sauer zur genannten aromatischen Dicarbonsäure verseift wird.

Die genannten Verfahren verlaufen mit schlechter Ausbeute und beruhen auf der Verwendung von schlecht zugänglichen geminalen Dichloriden bzw. von hochtoxischen Zwischenprodukten und sind daher für eine technische Anwendung wenig geeignet. Die Verfahren sind zudem hinsichtlich ihrer Anwendungsbreite auf homologe Verbindungen stark eingeschränkt.

Das erfindungsgemäße Verfahren stellt nunmehr eine verbesserte und breit anwendbare Methode zur Herstellung von aromatischen Dicarbonsäuren zur Verfügung. Es geht aus von den in technischen Mengen verfügbaren Bisphenolen.

Die Erfindung betrifft ein Verfahren zur Herstellung von aromatischen Dicarbonsäuren der Formel

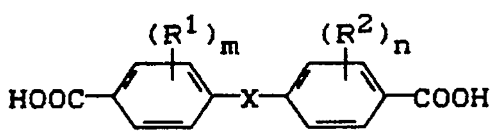

(I),

in der

X eine Einfachbindung, $C_1$-$C_{12}$-Alkylen, $C_2$-$C_{12}$-Alkyliden, $C_5$-$C_{15}$-Cycloalkylen, $C_5$-$C_{15}$-Cycloalkyliden, Sauerstoff, Schwefel, die Sulfongruppe, die Gruppe

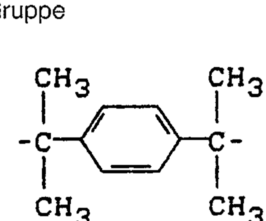

oder die Gruppe

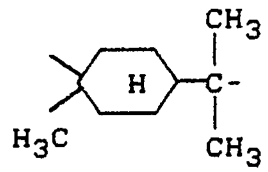

bedeutet, $R^1$ und $R^2$ unabhängig voneinander geradkettiges oder verzweigtes $C_1$-$C_4$-Alkyl oder $C_1$-$C_4$-Alkoxy bedeuten und
m und n unabhängig voneinander für ganze Zahlen von 0 bis 4 stehen,
das dadurch gekennzeichnet ist, daß man
a) ein Bisphenol der Formel

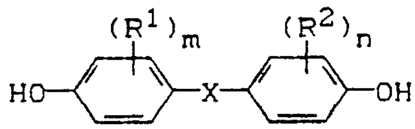 (II),

in der
$R^1$, $R^2$, m, n und X die genannte Bedeutung haben,
mit einem Sulfonsäurederivat der Formel
$R^3$-$SO_2$-$R^4$ (III),
in der
$R^3$ für geradkettiges oder verzweigtes $C_1$-$C_{12}$-Alkyl, $C_3$-$C_8$-Cycloalkyl, $C_6$-$C_{12}$-Aryl oder $C_7$-$C_{10}$-Aralkyl steht und
$R^4$ OH, Cl, Br oder O-$O_2$S-$R^3$ ist,
zu einem Bissulfonat der Formel

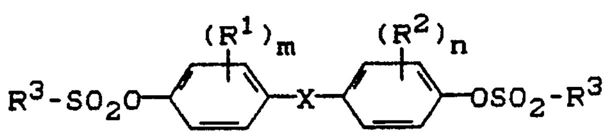 (IV),

in der
$R^1$, $R^2$, $R^3$, m, n und X die genannte Bedeutung haben,
umsetzt,
b) das erhaltene Bissulfonat in gelöster Form bei einem $H_2$-Druck von 1 bis 100 bar, bevorzugt 1 bis 50 bar, an einem trägerhaltigen oder trägerfreien Hydrierkatalysator in einer Menge von 0,5 bis 10 g, bevorzugt 2 bis 8 g, hydrieraktiver Komponente pro Mol Bissulfonat zum zugehörigen Kohlenwasserstoff der Formel

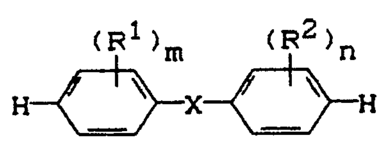 (V),

in der
$R^1$, $R^2$, X, m und n die genannte Bedeutung haben,
hydriert und
c) den erhaltenen Kohlenwasserstoff in bekannter Weise zweifach acyliert und die Acylgruppen zu Carboxylgruppen oxidiert.

$C_1$-$C_4$-Alkyl ist beispielsweise Methyl, Ethyl, Propyl, Isopropyl, Butyl, Isobutyl oder tert.-Butyl. Geradkettiges oder verzweigtes $C_1$-$C_{12}$-Alkyl bedeutet über das genannte $C_1$-$C_4$-Alkyl hinaus Amyl, Hexyl, Octyl, Decyl oder Dodecyl. $C_3$-$C_8$-Cycloalkyl ist beispielsweise Cyclopropyl, Cyclopentyl, Cyclohexyl, Cyclooctyl, Methylcyclopentyl, Methylcyclohexyl und ähnliche.

$C_1$-$C_4$-Alkoxy ist beispielsweise Methoxy, Ethoxy, Propoxy, Isopropoxy, Butoxy, Isobutoxy oder tert.-

Butoxy.

$C_1$-$C_{12}$-Alkylen ist beispielsweise Methylen, Dimethylen, Trimethylen, Tetramethylen, Pentamethylen, Hexamethylen, Octamethylen, Dodecamethylen, aber auch verzweigte Species, wie 1,2-Propylen, 1,2- und 1,3-Butylen und ähnliche.

$C_2$-$C_{12}$-Alkyliden ist beispielsweise Ethyliden, 1,1-Propyliden, 2,2-Propyliden, 1,1- oder 2,2-Butyliden, die entsprechenden Hexylidene, Octylidene oder Dodecanylidene.

$C_5$-$C_{15}$-Cycloalkylen ist beispielsweise 1,2- oder 1,3-Cyclopentylen, 1,2-, 1,3- und 1,4-Cyclohexylen, die entsprechenden Abkömmlinge von größeren Ringen sowie die zugehörigen Alkyl-substituierten Gruppen, wie 1,2- oder 1,3-Methylcyclopentanyl, 1,2-, 1,3- und 1,4-Methylcyclohexanyl, -Dimethyl-cyclohexanyl, -Trimethyl-cyclohexanyl und -Tetramethyl-cyclohexanyl sowie die entsprechenden Abkömmlinge größerer Ringe oder Ethyl-, Propyl- und Butyl-substituierte Gruppen dieser Art.

$C_5$-$C_{15}$-Cycloalkyliden entspricht im Bedeutungsumfang dem beschriebenen $C_5$-$C_{15}$-Cycloalkylen mit dem Unterschied, daß die zu den beiden aromatischen Kernen hinführenden Bindungen vom selben Ring-Kohlenstoff ausgehen.

$C_6$-$C_{12}$-Aryl bedeutet Phenyl, p-Tolyl, Naphthyl, Biphenylyl, bevorzugt Phenyl oder p-Tolyl.

$C_7$-$C_{10}$-Aralkyl ist beispielsweise Benzyl, Phenylethyl, Phenylpropyl oder Phenylbutyl, bevorzugt Benzyl.

Bevorzugte Bisphenole für das erfindungsgemäße Verfahren sind solche der Formel

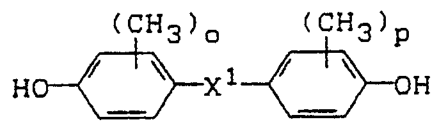 (VI),

in der

$X^1$ Methylen, $C_2$-$C_{12}$-Alkyliden oder $C_5$-$C_{10}$-Cycloalkyliden bedeuten und

o und p unabhängig voneinander für 0, 1 oder 2 stehen.

Beispiele für erfindungsgemäß einsetzbare Bisphenole sind: 4,4'-Dihydroxy-biphenyl, Bis-(4-hydroxyphenyl)-methan, 1,2-Bis-(4-hydroxyphenyl)-ethan, 1,3-Bis-(4-hydroxyphenyl)-propan, 1,7-Bis-(4-hydroxyphenyl)-heptan, 1,1-Bis-(4-hydroxyphenyl)-ethan, 2,2-Bis-(4-hydroxyphenyl)-propan, 1,1-Bis-(4-hydroxyphenyl)-butan, 1,1-Bis-(4-hydroxyphenyl)-propan, 2,2-Bis-(4-hydroxyphenyl)-butan, 1,1-Bis-(4-hydroxyphenyl)-2-methylpropan, 2,2-Bis-(4-hydroxyphenyl)-pentan, 3,3-Bis-(4-hydroxyphenyl)-pentan, 2,2-Bis-(4-hydroxyphenyl)-3-methylbutan, 2,2-Bis-(4-hydroxyphenyl)-4-methylpentan, 1,1-Bis-(4-hydroxyphenyl)-heptan, 4,4-Bis-(4-hydroxyphenyl)-heptan, 3,3-Bis-(4-hydroxyphenyl)-5-methylheptan, 1,1-Bis-(4-hydroxyphenyl)-2-ethylhexan, 4,4-Bis-(4-hydroxyphenyl)-2,6-dimethylheptan, 2,2-Bis-(4-hydroxyphenyl)-undecan, 1,3-Bis-(4-hydroxyphenyl)-cyclopentan, 1,3-Bis-(4-hydroxyphenyl)-cyclohexan, 1,4-Bis-(4-hydroxyphenyl)-cyclohexan, 1,1-Bis-(4-hydroxyphenyl)-cyclopentan, 1,1-Bis-(4-hydroxyphenyl)-cyclohexan, 1,1-Bis-(4-hydroxyphenyl)-2-methylcyclohexan, 1,1-Bis-(4-hydroxyphenyl)-3-methylcyclohexan, 1,1-Bis-(4-hydroxyphenyl)-3,5,5-trimethylcyclohexan, 1,1-Bis-(4-hydroxyphenyl)-cyclododecan, 2,2-Bis-(3,5-dimethyl-4-hydroxyphenyl)-propan, 4,4'-Dihydroxy-biphenylether, 4,4'-Dihydroxy-biphenylsulfid.

Bevorzugte Sulfonsäurederivate sind solche der Formel

$R^{13}$-$SO_2Cl$ (VII),

in der

$R^{13}$ geradkettiges oder verzweigtes $C_1$-$C_4$-Alkyl, bevorzugt Methyl, bedeutet.

Zur Hydrierung der Bissulfonate in gelöster Form werden als Lösungsmittel 10 bis 100 Gew.-Teile, bevorzugt 15 bis 50 Gew.-Teile, besonders bevorzugt 15 bis 20 Gew.-Teile pro Gew.-Teil des Bissulfonats eines $C_3$-$C_8$-Ketons, eines $C_3$-$C_8$-Esters, eines $C_4$-$C_8$-Ethers, eines $C_2$-$C_8$-Nitrils oder Pyridin oder ein Gemisch von ihnen eingesetzt $C_3$-$C_8$-Ketone sind beispielsweise Aceton, Methylethylketon, Methyl-propylketon, Methyl-butylketon, Dimethylketon und andere. $C_3$-$C_8$-Ester sind beispielsweise Methylacetat, Ethylacetat, Butylacetat, Methyl-butyrat, Methyl-benzoat und andere. $C_4$-$C_8$-Ether sind beispielsweise Diethylether, Methyl-tert.-butylether, Tetrahydrofuran, Dioxan, Dibutylether und andere. $C_2$-$C_8$-Nitrile sind beispielsweise Acetonitril, Propionitril, Butyronitril, Benzonitril und andere. Bis zu 50 % der Gesamtmenge dieses Lösungsmittels können durch einen $C_1$-$C_4$-Alkohol ersetzt sein, beispielsweise durch Methanol, Ethanol, Propanol oder Butanol.

Der $H_2$-Druck beträgt erfindungsgemäß 1 bis 100 bar, bevorzugt 1 bis 50 bar.

Es wird an einem trägerhaltigen oder trägerfreien Hydrierkatalysator in einer Menge von 0,5 bis 10 g, bevorzugt 2 bis 8 g, hydrieraktiver Komponente pro Mol des Bissulfonats hydriert. Die hydrieraktive

Komponente kann ein Platinmetall, wie Platin, Palladium oder Ruthenium, bevorzugt Palladium, oder ein unedles Metall wie Nickel, beispielsweise als Raney-Nickel, sein. Als Träger kommen A-Kohle $SiO_2$, $Al_2O_3$, Bimsstein und andere in Betracht Sowohl die hydrieraktiven Komponenten als auch die Träger sind dem Fachmann bekannt. In bevorzugter Form wird ein Platin- oder Palladium-Trägerkatalysator, besonders bevorzugt ein Palladium/Kohle-Katalysator (0,5-10 Gew.-% Pd, bevorzugt 2-8 Gew.-% Pd) eingesetzt.

Die Hydrierung erfolgt in bevorzugter Weise so, daß die Menge an Katalysator portionsweise oder kontinuierlich im Verlaufe der Hydrierung zugesetzt wird.

Acylierungsmittel sind Säurechloride oder Säureanhydride, wie Acetylchlorid, Acetanhydrid, Propionylchlorid, Butyrylchlorid und weitere in Gegenwart von Friedel-Crafts-Katalysatoren, wie Aluminiumchlorid, Eisenchlorid und anderen. Solche Acylierungsmittel und Friedel-Crafts-Katalysatoren sind dem Fachmann bekannt.

Die Oxidation der Acylgruppe zur Carboxylgruppe kann beispielsweise mit einem Hypochlorit oder Hypobromit des Kaliums, Natriums oder Calciums, bevorzugt mit Natriumhypochlorit-Lösung (Bleichlauge), in grundsätzlich bekannter Weise erfolgen.

Als erfindungsgemäß herstellbare Säuren seien beispielsweise genannt: 2,2-Bis-(4-carboxyphenyl)-4-methylpentan, 3,3-Bis-(4-carboxyphenyl)-5-methylheptan, 1,7-Bis-(4-carboxyphenyl)-heptan, 1,3-Bis-(4-carboxyphenyl)-cyclopentan, 1,3-Bis-(4-carboxyphenyl)-cyclohexan, 1,4-Bis-(4-carboxyphenyl)-cyclohexan, Bis-(3,5-dimethyl-4-carb oxyphenyl)-methan, 2,2-Bis-(3,5-dimethyl-4-carboxyphenyl)-propan, 1,1-Bis-(4-carboxyphenyl)-cyclopentan, 1,1-Bis-(4-carboxyphenyl)-cyclohexan, 1,1-Bis-(4-carboxyphenyl)-3-methylcyclohexan, 1,1-Bis-(4-carboxyphenyl)-3,5,5-trimethylcyclohexan, 1,1-Bis-(4-carboxyphenyl)-cyclododecan.

Das erfindungsgemäße Verfahren sei am Beispiel des 2,2=Bis-(4-hydroxyphenyl)-propan ("Bisphenol A") unter Verwendung von Methansulfonsäurechlorid, von Acetylchlorid und von Natriumhypochlorit wie folgt formelmäßig dargestellt:

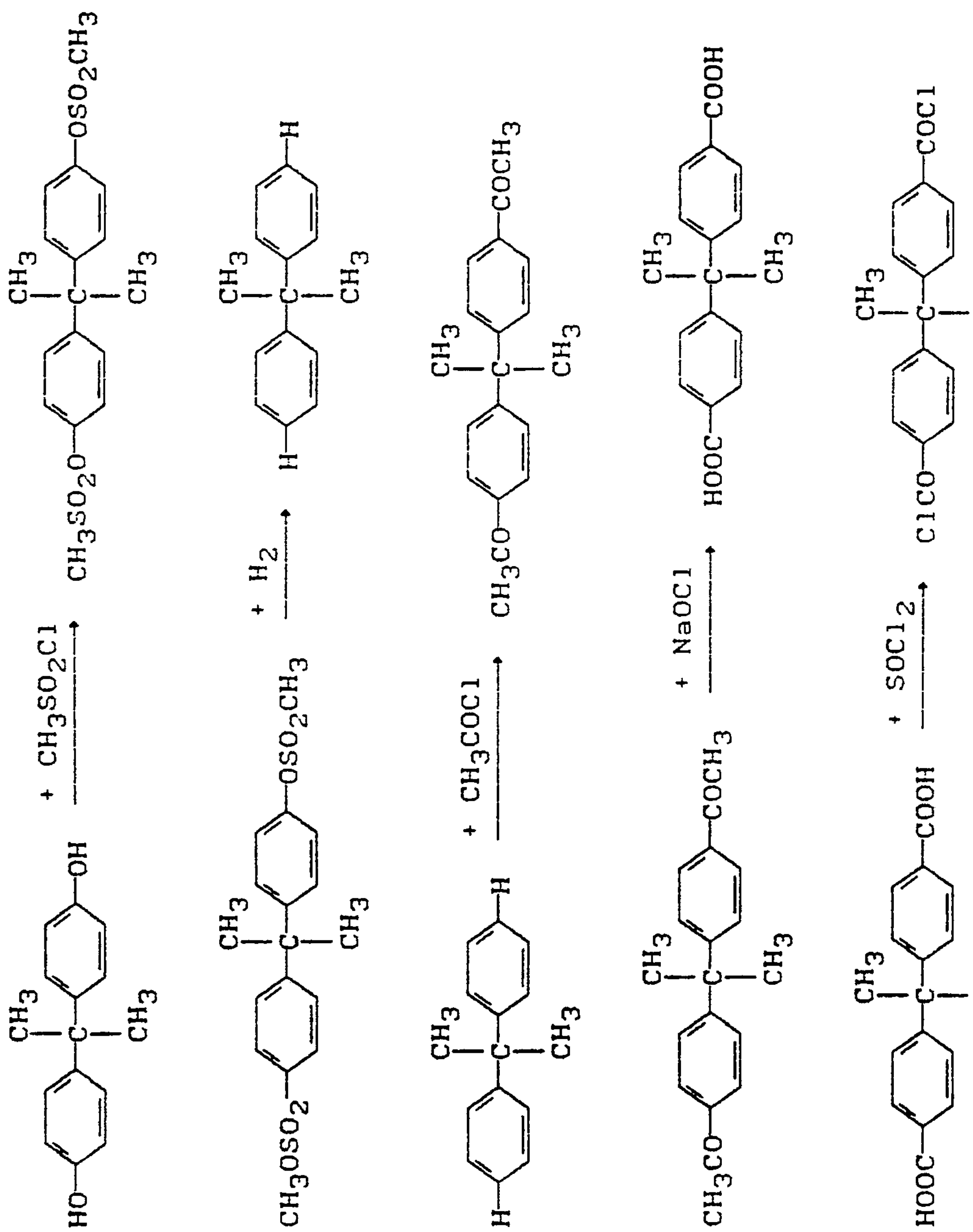


Verbindung A

Verbindung B

Verbindung C

Verbindung D

Verbindung E

Aus den erfindungsgemäß herstellbaren Säuren können in bekannter Weise die Säurechloride (beispielsweise durch Umsetzung mit Thionylchlorid) oder die Ester (beispielsweise durch direkte Veresterung der Säure mit Alkoholen, wie Methanol oder Ethanol oder durch Umsetzung der Säurechloride mit solchen Alkoholen) hergestellt werden, die häufig günstiger zu Polymeren umgesetzt werden können als die freien Säuren.

Viele der erfindungsgemäß herstellbaren Säuren sowie die daraus herstellbaren Dichloride und die Di-

$C_1$-$C_4$-alkylester dieser Dicarbonsäuren sind neu. Die Erfindung betrifft daher weiterhin aromatische Dicarbonsäuren der Formel

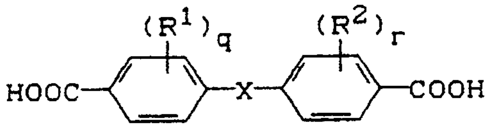 (VIII),

in der
X eine Einfachbindung, $C_1$-$C_{12}$-Alkylen, $C_2$-$C_{12}$-Alkyliden, $C_5$-$C_{15}$-Cycloalkylen, $C_5$-$C_{15}$-Cycloalkyliden, Sauerstoff, Schwefel, die Sulfongruppe oder die Gruppe

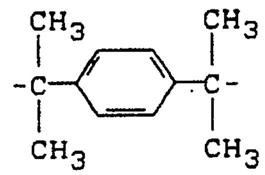

oder die Gruppe

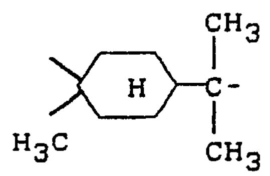

bedeuten,
$R^1$ und $R^2$ unabhängig voneinander geradkettiges oder verzweigtes $C_1$-$C_4$-Alkyl bedeuten und
q und r unabhängig voneinander für ganze Zahlen von 0 bis 4 stehen, wobei mindestens einer der Indices q und r verschieden von 0 ist, wenn X eine Einfachbindung, $C_1$-$C_6$-Alkylen, lineares $C_2$-$C_4$-Alkyliden, lineares $C_7$-Alkyliden, 2,2-Propyliden, Sauerstoff, Schwefel, die Sulfongruppe oder die Gruppe

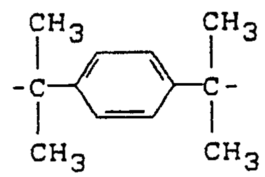

ist,
sowie die Dichloride und die Di-$C_1$-$C_4$-alkylester dieser Dicarbonsäuren, bevorzugt die Dicarbonsäuren selbst.

Unter den neuen aromatischen Dicarbonsäuren der Formel (VI) seien bevorzugt genannt: 2,2-Bis-(3,5-dimethyl-4-carboxyphenyl)-propan, 1,1-Bis-(4-carboxyphenyl)-cyclohexan, 1,1-Bis-(4-carboxyphenyl)-3-methyl-cyclohexan und 1,1-Bis-(4-carboxyphenyl)-3,5,5-trimethyl-cyclohexan.

Die Erfindung betrifft weiterhin ein Verfahren zur Herstellung der aromatischen Kohlenwasserstoffe der obigen Formel (V) als Zwischenprodukte, das dadurch gekennzeichnet ist, daß man

a) ein Bisphenol der obigen Formel (II) mit einem Sulfonsäurederivat der obigen Formel (III) zu einem Bissulfonat der obigen Formel (IV) umsetzt und

b) das Bissulfonat (IV) in gelöster Form bei einem $H_2$-Druck von 1-100 bar, bevorzugt 1-50 bar, an einem trägerhaltigen oder trägerfreien Hydrierkatalysator in einer Menge von 0,5-10 g, bevorzugt 2-8 g, hydrieraktiver Komponente pro Mol Bissulfonat (IV) hydriert.

Die Erfindung betrifft auch die neuen aromatischen Kohlenwasserstoffe der Formel

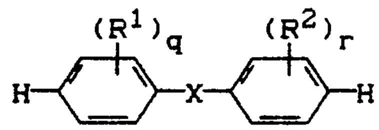

(IX)

in der $R^1$, $R^2$, X, q und r die obige Bedeutung haben.

Unter den neuen aromatischen Kohlenwasserstoffen (IX) seien bevorzugt genannt: 2,2-Bis-(3,5-dimethylphenyl)-propan, 1,1-Diphenyl-cyclohexan, 1,1-Diphenyl-3-methyl-cyclohexan und 1,1-Diphenyl-3,5,5-trimethyl-cyclohexan.

## Beispiele

Das im folgenden am Beispiel des 2,2-Bis-(4-hydroxyphenyl)-propans ausführlich beschriebene Verfahren ist ohne wesentliche Änderungen auf alle weiteren Bisphenole anwendbar:

a) 2,2-Bis-(4-methylsulfonyloxy-phenyl)-propan (Verbindung A):

2 Mol 2,2-Bis-(4-hydroxyphenyl)-propan wurden unter Zusatz von 4 Mol Triethylamin in Methylenchlorid mit 4,02 Mol Methansulfonsäurechlorid bei Raumtemperatur umgesetzt. Nach wäßriger Aufarbeitung und Kristallisation aus Isopropanol erhielt man 83 % Bissulfonat,
Fp.: 115 - 116° C,

| | | | |
|---|---|---|---|
| Analyse $C_{17}H_{20}O_6S_2$ ber.: | C 53,1, | H 5,2, | S 16,6, |
| gef.: | C 53,1, | H 5,2, | S 16,6; |

analog:
1,1-Bis-(4-methylsulfonyloxy-phenyl)-cyclohexan (81 %):
Fp.: 114-115° C,

| | | | |
|---|---|---|---|
| Analyse $C_{20}H_{24}O_6S_2$ ber.: | C 56,6, | H 5,7, | S 15,1, |
| gef.: | C 56,5, | H 5,7, | S 15,1; |

analog:
1,1-Bis-(4-methansulfonyloxy-phenyl)-3,5,5-trimethylcyclohexan (78 %):
Fp.: 129-131° C,

| | | | |
|---|---|---|---|
| Analyse $C_{23}H_{30}O_6S_2$ ber.: | C 59,2, | H 6,4, | S 13,7, |
| gef.: | C 59,2, | H 6,4, | S 13,7. |

b) 2,2-Diphenylpropan (Verbindung B):

0,5 Mol der Verbindung A wurden in 3.000 ml Essigester unter Zusatz von 27 g Palladium/Kohle (5 % Pd) und 1 Mol Triethylamin bei 40° C und 20 bar $H_2$ gerührt; im Verlauf der Reaktion wurden noch insgesamt 15 g Pd/C zugesetzt. Nach Destillation erhielt man 84 % der Verbindung B.

| | | |
|---|---|---|
| Analyse $C_{15}H_{16}$ ber.: | C 91,8, | H 8,2, |
| gef.: | C 91,8, | H 8,2; |

analog:

1,1-Diphenylcyclohexan (98 %):

Fp.: 47° C,

| | | |
|---|---|---|
| Analyse $C_{18}H_{20}$ ber.: | C 91,5, | H 8,5, |
| gef.: | C 90,6, | H 8,9, |

MS: m/z 236 (74 %); analog:
1,1-Diphenyl-3,5,5-trimethyl-cyclohexan (72 %):

| | | |
|---|---|---|
| Analyse $C_{21}H_{26}$ ber.: | C 90,6, | H 9,4, |
| gef.: | C 89,9, | H 9,1, |

MS: m/z 278 (92 %).

c) 2,2-Bis-(4-acetyl-phenyl)-propan (Verbindung C):

Zu einer Lösung von 1,9 Mol Aluminiumchlorid und 1,7 Mol Acetylchlorid in 2 l Ethylenchlorid wurden bei 10° C 0,8 Mol der Verbindung B in 300 ml Ethylenchlorid getropft und anschließend 15 Stunden bei Raumtemperatur nachgerührt. Nach wäßriger Aufarbeitung wurde über eine mit Kieselgel gefüllte Fritte mit Petrolether/Essigester filtriert. Nach Abtrennung eines Vorlaufs erhielt man 75 % der Verbindung C. MS: m/z 280 (35 %).
Fp.: 73° C,
$^1$H-NMR (200 MHz, $CDCl_3$): $\delta$ = 7,88 (d, 4H):
7,30 (d, 4H);
2,58 (s, 6H);
1,72 (s, 6H). analog:
1,1-Bis-(4-acetyl-phenyl)-cyclohexan (69 %):
Fp.: 110-113° C,

| | | |
|---|---|---|
| Analyse $C_{22}H_{24}O_2$ ber.: | C 82,5, | H 7,5, |
| gef.: | C 81,8, | H 7,6 |

MS: m/z 320 (40 %);
analog:
1,1-Bis-(4-acetyl-phenyl)-3,5,5-trimethyl-cyclohexan (89 %):

| | | |
|---|---|---|
| Analyse $C_{25}H_{30}O_2$ ber.: | C 82,9, | H 8,3, |
| gef.: | C 82,9, | H 8,4, |

MS: m/z 362 (29 %).

d) 2,2-Bis-(4-carboxyphenyl)-propan (Verbindung D):

Zu 1,5 l Chlorlauge (1,85 Mol/kg) und 100 g NaOH tropfte man bei 20 bis 25° C eine Lösung von 0,35 Mol der Verbindung C in 400 ml Dioxan. Nach 24 Stunden wurde die Lösung mit Methylenchlorid gewaschen und die Dicarbonsäure D durch Ansäuern und Umfällen aus NaOH isoliert (85 %).
Fp.: >320° C; IR (KBr): 2973, 1688, 1607,1386, 1284 $cm^{-1}$
analog:
1,1-Bis-(4-carboxyphenyl)-cyclohexan (85 %):
Fp.: >320° C,
IR (KBr): 2938, 2862, 1691, 1284, 1606, 1386 $cm^{-1}$;
analog:
1,1-Bis-(4-hydroxyphenyl)-3,5,5-trimethylcyclohexan (72 %):
Fp.: >320° C,
IR (KBr): 2952, 2927, 1692, 1607, 1388, 1283 $cm^{-1}$.

e) 2,2-Bis-(4-chlorcarbonylphenyl)-propan (Verbindung E):

0,5 Mol D wurden mit einem Überschuß Thionylchlorid (250 ml) übergossen, mit einigen Tropfen Pyridin versetzt und bis zum Ende der HCl-Entwicklung zum Rückfluß erhitzt. überschüssiges Thionylchlorid wurde abdestilliert und der Rückstand im Hochvakuum destilliert oder aus Leichtbenzin umkristallisiert. Man erhielt 73 % Säurechlorid.
Kp. (0,15 mm): 198° C,

| | | | |
|---|---|---|---|
| Analyse $C_{17}H_{14}Cl_2O_2$ ber.: | C 63,5, | H 4,4, | Cl 22,1, |
| gef.: | C 63,6, | H 4,5, | Cl 21,6; |

analog:
1,1-Bis-(4-chlorcarbonylphenyl)-cyclohexan (63 %):
GC-MS: 99,5 %, m/z 360/362/364 (14, 10, 1 %);
analog:
1,1-Bis-(4-chlorcarbonylphenyl)-3,5,5-trimethylcyclohexan (74 %):
GC-MS: 98,9 % m/z 402/404/406 (8,5, 5,6, 1 %).

f) 2,2-Bis-(4-methoxycarbonylphenyl)-propan

0,5 mol 2,2-Bis-(4-chlorcarbonyl-phenyl)-propan wurden in 500 ml abs. Methanol unter Zusatz von 2 g Triethylamin gerührt, bis die HCl-Entwicklung beendet war. Der nach Eindampfen verbleibende Rückstand wurde aus wenig Methanol umkristallisiert (Ausb. 95 %)

| | | |
|---|---|---|
| Analyse: ber.: | C 73,1 %; | H 6,5 % |
| gef.: | C 73,1 %, | H 6,4 % |

$^1$H-NMR (200 MHz, $CDCl_3$): d = 7,9 (4H); 7,3 (4H); 3,9 (6H); 1,7 (6H) ppm

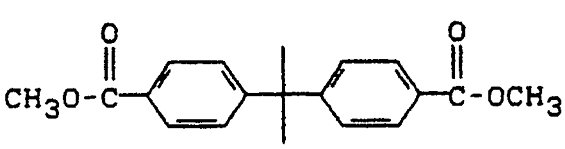

**Ansprüche**

1. Verfahren zur Herstellung von aromatischen Dicarbonsäuren der Formel

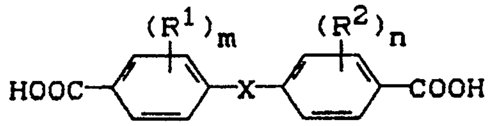

,

in der

X eine Einfachbindung, $C_1$-$C_{12}$-Alkylen, $C_2$-$C_{12}$-Alkyliden, $C_5$-$C_{15}$-Cycloalkylen, $C_5$-$C_{15}$-Cycloalkyliden, Sauerstoff, Schwefel, die Sulfongruppe, die Gruppe

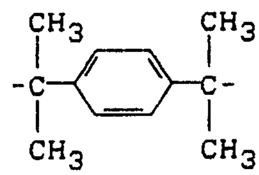

oder die Gruppe

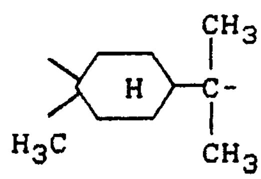

bedeuten,

$R^1$ und $R^2$ unabhängig voneinander geradkettiges oder verzweigtes $C_1$-$C_4$-Alkyl oder $C_1$-$C_4$-Alkoxy bedeuten und

m und n unabhängig voneinander für ganze Zahlen von 0 bis 4 stehen,

dadurch gekennzeichnet, daß

a) ein Bisphenol der Formel

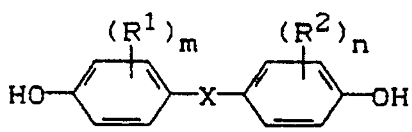

,

in der

$R^1$, $R^2$, m, n und X die genannte Bedeutung haben,

mit einem Sulfonsäurederivat der Formel

$R^3$-$SO_2$-$R^4$ ,

in der

$R^3$ für geradkettiges oder verzweigtes $C_1$-$C_{12}$-Alkyl, $C_3$-$C_8$-Cycloalkyl, $C_6$-$C_{12}$-Aryl oder $C_7$-$C_{10}$-Aralkyl steht und

$R^4$ OH, Cl, Br oder O-$O_2$S-$R^3$ ist,

zu einem Bissulfonat der Formel

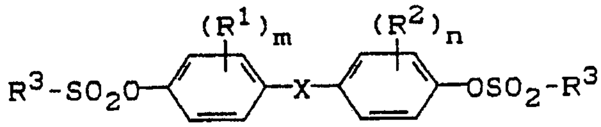

,

in der
$R^1$, $R^2$, $R^3$, m, n und X die genannte Bedeutung haben,
umsetzt,
b) das erhaltene Bissulfonat in gelöster Form bei einem $H_2$-Druck von 1 bis 100 bar, bevorzugt 1 bis 50 bar, an einem trägerhaltigen oder trägerfreien Hydrierkatalysator in einer Menge von 0,5 bis 10 g, bevorzugt 2 bis 8 g, hydrieraktiver Komponente pro Mol Bissulfonat zum zugehörigen Kohlenwasserstoff der Formel

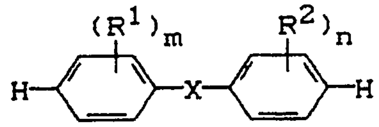
,

in der
$R^1$, $R^2$, X, m und n die genannte Bedeutung haben,
hydriert und
c) den erhaltenen Kohlenwasserstoff in bekannter Weise zweifach acyliert und die Acylgruppen zu Carboxylgruppen oxidiert.

2. Verfahren nach Anspruch 1, dadurch gekennzeichnet, daß die Hydrierung in einem Losungsmittel aus der Gruppe der $C_3$-$C_8$-Ketone, der $C_3$-$C_8$-Ester, der $C_4$-$C_8$-Ether, der $C_2$-$C_8$-Nitrile und Pyridin in einer Menge von 10 bis 100 Gew.-Teilen pro Gew.-Teil des Bissulfonats, bevorzugt 15 bis 50 Gew.-Teile, besonders bevorzugt 15 bis 20 Gew.-Teile, durchgeführt wird, wobei bis zu 50 % der Gesamtmenge des Lösungsmittels durch einen $C_1$-$C_4$-Alkohol ersetzt sein kann.

3. Verfahren nach Anspruch 1, dadurch gekennzeichnet, daß der Hydrierkatalysator ein Trägerkatalysator mit 0,5 bis 10 Gew.-% Platinmetall ist.

4. Verfahren nach Anspruch 1, dadurch gekennzeichnet, daß der Hydrierkatalysator portionsweise oder kontinuierlich im Verlaufe der Hydrierung zugesetzt wird.

5. Aromatische Dicarbonsäuren der Formel

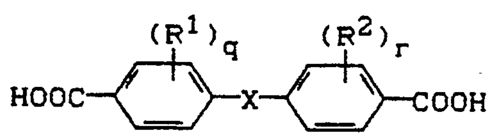
,

in der X eine Einfachbindung, $C_1$-$C_{12}$-Alkylen, $C_2$-$C_{12}$-Alkyliden, $C_5$-$C_{15}$-Cycloalkylen, $C_5$-$C_{15}$-Cycloalkyliden, Sauerstoff, Schwefel, die Sulfongruppe, die Gruppe

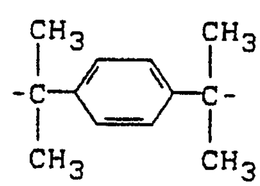

oder die Gruppe

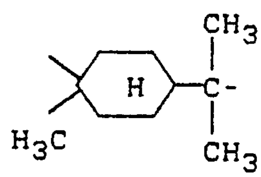

bedeuten,
$R^1$ und $R^2$ unabhängig voneinander geradkettiges oder verzweigtes $C_1$-$C_4$-Alkyl bedeuten und
q und r unabhängig voneinander für ganze Zahlen von 0 bis 4 stehen, wobei mindestens einer der Indices q und r verschieden von 0 ist, wenn X eine Einfachbindung, $C_1$-$C_6$-Alkylen, lineares $C_2$-$C_4$-Alkyliden,

lineares $C_7$-Alkyliden, 2,2-Propyliden, Sauerstoff, Schwefel, die Sulfongruppe oder die Gruppe

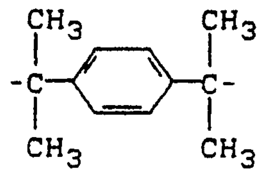

ist,
sowie die Dichloride und die Di-$C_1$-$C_4$-alkylester dieser Dicarbonsäuren.

6. Aromatische Dicarbonsäuren aus der Gruppe 2,2-Bis-(3,5-dimethyl-4-carboxyphenyl)-propan, 1,1-Bis-(4-carboxyphenyl)-cyclohexan, 1,1-Bis-(4-carboxyphenyl)-3-methyl-cyclohexan und 1,1-Bis-(4-carboxyphenyl )-3,5,5-trimethyl-cyclohexan.

7. Verfahren zur Herstellung von aromatischen Kohlenwasserstoffen der Formel

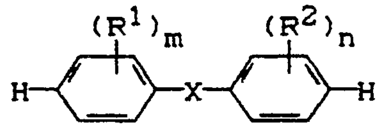

,

in der
X eine Einfachbindung, $C_1$-$C_{12}$-Alkylen, $C_2$-$C_{12}$-Alkyliden, $C_5$-$C_{15}$-Cycloalkylen, $C_5$-$C_{15}$-Cycloalkyliden, Sauerstoff, Schwefel, die Sulfongruppe, die Gruppe

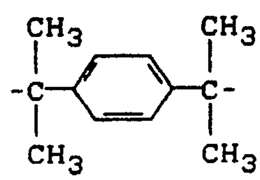

oder die Gruppe

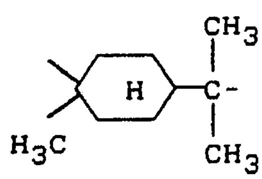

bedeuten,
$R^1$ und $R^2$ unabhängig voneinander geradkettiges oder verzweigtes $C_1$-$C_4$-Alkyl oder $C_1$-$C_4$-Alkoxy bedeuten und
m und n unabhängig voneinander für ganze Zahlen von 0 bis 4 stehen,
dadurch gekennzeichnet, daß

a) ein Bisphenol der Formel

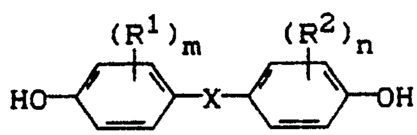

,

in der
$R^1$, $R^2$, m, n und X die genannte Bedeutung haben,
mit einem Sulfonsäurederivat der Formel
$R^3$-$SO_2$-$R^4$ ,

in der
$R^3$ für geradkettiges oder verzweigtes $C_1$-$C_{12}$-Alkyl, $C_3$-$C_8$-Cycloalkyl, $C_6$-$C_{12}$-Aryl oder $C_7$-$C_{10}$-Aralkyl steht und
$R^4$ OH, Cl, Br oder O-$O_2$S-$R^3$ ist,
zu einem Bissulfonat der Formel

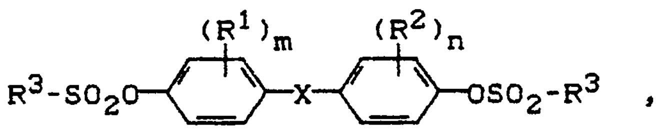

in der
$R^1$, $R^2$, $R^3$, m, n und X die genannte Bedeutung haben,
umsetzt und

b) das erhaltene Bissulfonat in gelöster Form bei einem $H_2$-Druck von 1 bis 100 bar, bevorzugt 1 bis 50 bar, an einem trägerhaltigen oder trägerfreien Hydrierkatalysator in einer Menge von 0,5 bis 10 g, bevorzugt 2 bis 8 g, hydrieraktiver Komponente pro Mol Bissulfonat hydriert.

8. Aromatische Kohlenwasserstoffe der Formel

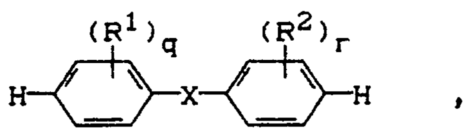

in der
X eine Einfachbindung, $C_1$-$C_{12}$-Alkylen, $C_2$-$C_{12}$-Alkyliden, $C_5$-$C_{15}$-Cycloalkylen, $C_5$-$C_{15}$-Cycloalkyliden, Sauerstoff, Schwefel, die Sulfongruppe, die Gruppe

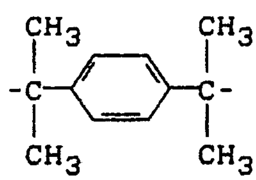

oder die Gruppe

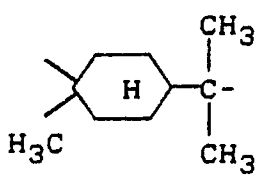

bedeuten,
$R^1$ und $R^2$ unabhängig voneinander geradkettiges oder verzweigtes $C_1$-$C_4$-Alkyl bedeuten und
q und r unabhängig voneinander für ganze Zahlen von 0 bis 4 stehen, wobei mindestens einer der Indices q und r verschieden von 0 ist, wenn X eine Einfachbindung, $C_1$-$C_6$-Alkylen, lineares $C_2$-$C_4$-Alkyliden, lineares $C_7$-Alkyliden, 2,2-Propyliden, Sauerstoff, Schwefel, die Sulfongruppe oder die Gruppe

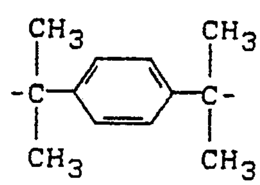

ist.

9. Die aromatischen Kohlenwasserstoffe 2,2-Bis- (3,5-dimethyl-phenyl)-propan, 1,1-Diphenyl-cyclohexan, 1,1-Diphenyl-3-methyl-cyclohexan und 1,1-Diphenyl-3,5,5-trimethyl-cyclohexan.